Europäisches Patentamt

European Patent Office

Office européen des brevets

(11) Veröffentlichungsnummer: **0 411 204 B1**

# EUROPÄISCHE PATENTSCHRIFT

(45) Veröffentlichungstag der Patentschrift: **30.11.94**

(51) Int. Cl.5: **G01N  33/28**

(21) Anmeldenummer: **89117831.1**

(22) Anmeldetag: **27.09.89**

(54) **Verfahren zur Feststellung des Alkoholgehaltes und/oder des Heizwertes von Kraftstoffen.**

(30) Priorität: **01.07.89 DE 3921707**

(43) Veröffentlichungstag der Anmeldung:
**06.02.91 Patentblatt  91/06**

(45) Bekanntmachung des Hinweises auf die
Patenterteilung:
**30.11.94 Patentblatt  94/48**

(84) Benannte Vertragsstaaten:
**DE FR GB IT SE**

(56) Entgegenhaltungen:
EP-A- 377 782
GB-A- 2 055 472
US-A- 4 174 498
US-A- 4 288 741
US-A- 4 426 616

IEEE TRANS. VEHICULAR TECHN., Band VT-27, Nr. 3, August 1978, IEEE, New York, NY (US); J.W. HILE et al., Seiten 142-144&NUM;

PATENT ABSTRACTS OF JAPAN, Band 6, Nr. 133 (P-129)[1011], 20. Juli 1982&NUM;

(73) Patentinhaber: **FEV Motorentechnik GmbH & Co. KG**
**Neuenhofstrasse 181**
**D-52078 Aachen (DE)**

(72) Erfinder: **Schmitz, Günter, Dr.-Ing.**
**Zehnthofweg 31**
**D-5100 Aachen (DE)**
Erfinder: **Reggelin, Bernd, Dipl.-Ing.**
**Johannstrasse 34**
**D-5100 Aachen (DE)**

(74) Vertreter: **Langmaack, Jürgen, Dipl.-Ing. et al**
**Patentanwälte**
**Maxton . Maxton . Langmaack**
**Postfach 51 08 06**
**D-50944 Köln (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren zur Feststellung des Alkoholgehaltes und/oder des Heizwertes von Kraftstoffen durch Messung elektrisch meßbarer Größen in einer den Kraftstoff enthaltenden Meßzelle zur Bestimmung der Dielektrizitätszahl des in der Zelle enthaltenen Gemisches als Kenngröße des Alkoholgehaltes bzw. des Heizwertes.

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme bereitet dabei die laufende Feststellung des Alkoholgehaltes des der Brennkraftmaschine im Betrieb laufend zugeführten Kraftstoffs bei Fahrzeugmotoren, bei denen durch das beliebige Tanken der Kraftstoffarten jede mögliche Mischung erreicht werden kann.

Die bekannten optischen Verfahren sind zu diesem Zweck kaum geeignet, da sie meist Grenzflächeneffekte zur Bestimmung des Brechungsindexes ausnutzen, aus dem dann auf den Alkoholanteil geschlossen werden kann. Abgesehen von der Schwierigkeit der Auswertung bei Fahrzeugmotoren ist ein Nachteil dieses Verfahrens auch, daß die messend zu beobachtende Mischung eine hohe Homogenität aufweisen muß, die insbesondere auch an der Grenzfläche vorhanden sein muß. Mit diesem Verfahren wurden nicht die erforderlichen Genauigkeiten erreicht. Außerdem besteht im optischen Bereich eine große Abhängigkeit von dem Aromatengehalt der verwendeten Benzinkraftstoffe, so daß sich je nach Kraftstoffirma und Charge zusätzlich erhebliche Fehler einstellen.

Es wurde daher vorgeschlagen, den Alkoholteil in Kraftstoffen durch eine Dielektrizitätsbestimmung festzustellen, und ein solches Verfahren hätte den Vorteil, daß die Problematik der Messung von Grenzflächeneffekten behoben ist, da die Messung volumetrisch erfolgt. Andererseits geht in die volumetrische Dielektrizitätsbestimmung in starkem Maße der Leitwert der Mischung ein (Querempfindlichkeit). Da aber der Leitwert sehr stark von Verunreinigungen oder Wasseranteilen abhängt, führt auch ein solches Meßverfahren zu unbrauchbaren Ergebnissen.

In der Schrift "Proceedings of the Fourth International Symposium on Alcohol Fuels Technology", Sao Paulo, Brasilien, vom 05.10.1980, wird die Möglichkeit der Feststellung des Alkoholgehaltes von Kraftstoffen durch Dielektrizitätsmessungen beschrieben. Aufgrund der Einflüsse von Temperatur und Leitwert (hervorgerufen durch Wasseranteile oder andere Verschmutzungen im Kraftstoff) wurde das Verfahren jedoch verworfen, da eine für Verbrennungsmotoren geeignete, zuverlässige Messung nicht durchgeführt werden konnte.

Aus den Veröffentlichungen IEEE Trans. Vehicular Techn. Vol. VT-27(3), Aug. 1987, 142-144 sowie aus Patent Abstracts of Japan, 6(133) (P-129) (1011) ist es bekannt, die Dielektrizitätszahl über eine Kapazitätsbestimmung zu erfassen. Die Messungen nach dem vorbekannten Verfahren sind jedoch dann in hohem Maße fehlerbehaftet, wenn sich durch Verunreinigungen, Wasserbeimengung oder dergl. die Leitfähigkeit des zu untersuchenden Kraftstoffs unabhängig vom Alkoholgehalt verändert. Zuverlässige Messungen sind hiermit nicht durchführbar. In der als älteres Recht geltenden, nachveröffentlichten EP 0 377 782 A2 ist angegeben Kapazität und Leitwert zu messen und aus beiden Größen gemeinsam den Alkoholgehalt des zu untersuchenden Kraftstsoffs zu bestimmen.

Der Erfindung liegt daher die Aufgabe zugrunde, ein Verfahren der eingangs bezeichneten Art zu schaffen, das insbesondere in der Anwendung auf Fahrzeugmotoren eine genaue und zuverlässige Feststellung des Alkoholanteils und/oder des Heizwertes von Kraftstoffen ermöglicht.

Diese Aufgabe wird mit den im Anspruch 1 angegebenen Verfahrensschritten gelöst.

In vorteilhafter Ausgestaltung kann dabei die Messung der Kapazität durch Beaufschlagung der Meßzelle mit einer sinusförmigen Spannung und der Auswertung des durch die Meßzelle fließenden Stromes hinsichtlich des Imaginärteils erfolgen. Dies kann durch eine einfache Synchrongleichrichtung oder aber auch durch Multiplikation mit einer cos-förmigen Spannung gleicher Frequenz geschehen. Das sich ergebende Ausgangssignal ist dann proportional zur Kapazität.

Hinsichtlich weiterer vorteilhafter Merkmale der Erfindung wird auf die Ansprüche, die Zeichnungen und die nachfolgende Beschreibung Bezug genommen.

Ausführungsbeispiele der Erfindung werden anhand der Zeichnungen beschrieben.

Fig. 1 zeigt im Diagramm die Dielektrizitätszahl als Funktion des Leitwertes G in Abhängigkeit von dem Wassergehalt und dem Methanolgehalt des Kraftstoffes.

Fig. 2 zeigt ein bevorzugtes Beispiel einer Meßzelle zur Ausführung des Verfahrens gemäß der Erfindung.

Fig. 3 zeigt schematisch eine Anordnung zur Anwendung des Verfahrens gemäß der Erfindung zur Steuerung bzw. Regelung einer Einspritzbrennkraftmaschine.

Fig. 4 zeigt eine bevorzugte Ausführungsform einer Schaltung zur Bestimmung der Dielektrizitätszahl durch Kapazitätsmessung.

Fig. 5 zeigt eine bevorzugte Ausführungsform einer Schaltung, in der der Meßvorgang mit Hilfe unterschiedlicher Frequenzen erfolgt.

Fig. 6 zeigt eine weitere bevorzugte Ausführungsform, bei der die Messung in zwei Phasen erfolgt.

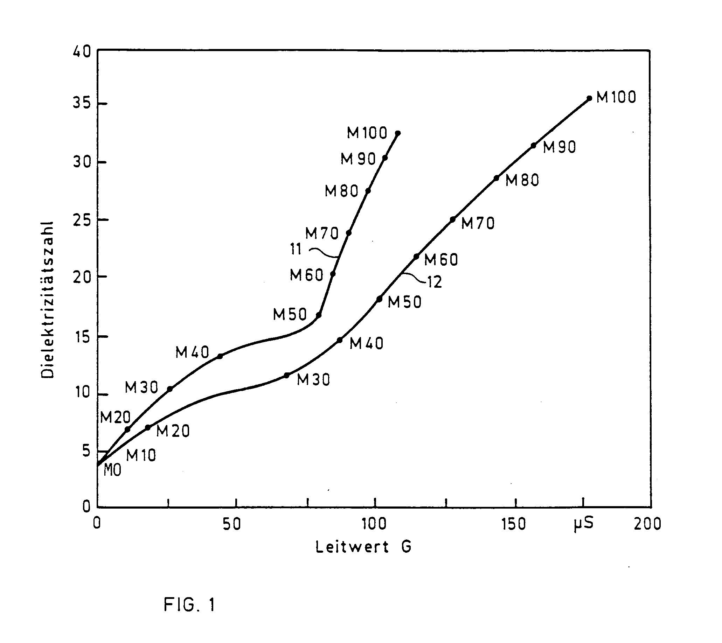

In der Darstellung gemäß Fig. 1 sind die Werte der Dielektrizitätsmessung in der Ordinate und die Leitwerte G in der Abszisse aufgetragen. Linienzug 11 zeigt die Abhängigkeit der Werte der Dielektrizitätsmessung von dem Anteil der Methanolbeimischung bei einem Wasseranteil von 0% im Kraftstoff, während Linienzug 12 die entsprechenden Werte bei einem Anteil von 2,5% Wasser darstellt. Auf den Linienzügen sind die jeweiligen Meßpunkte für variable Methanolanteile von 0% bis 100% (MO bis M100) aufgetragen.

Bei einer Kapazitätsmessung zur Bestimmung der Dielektrizitätszahl treten Schwierigkeiten bedingt durch den Einfluß verschiedener Leitfähigkeiten auf. In Fig. 1 ist erkennbar, welchen Einfluß ein bestimmter Wasseranteil auf den Leitwert hat. Stärkere Einflüsse auf den Leitwert werden auch bei Säuren und Salzen beobachtet, die schon in geringen Konzentrationen den Leitwert drastisch erhöhen können, ohne eine nennenswerte Kapazitätsänderung zu bewirken. In Schwingschaltungen zur Bestimmung der Kapazität wirken sich unterschiedliche Leitwerte jedoch sehr negativ aus; in RC-Schaltungen ergibt sich eine Fehlmessung der Kapazität, die nachträglich durch separate Messung des Leitwertes kompensiert werden muß, und in LC-Schaltungen ergeben sich ebenfalls Probleme hinsichtlich des Meßfehlers bis hin zu Problemen mit der grundsätzlichen Schwingfähigkeit der Schaltung.

Durch Meßschaltungen, die durch gezielte schaltungstechnische oder rechnerische Auswertung von mathematischen Zusammenhängen eine Unterdrückung des Leitwerteinflusses ermöglichen, ist über eine Kapazitätsbestimmung - versehen mit einer temperaturabhängigen Korrektur - eine genaue Bestimmung des Alkoholanteils möglich.

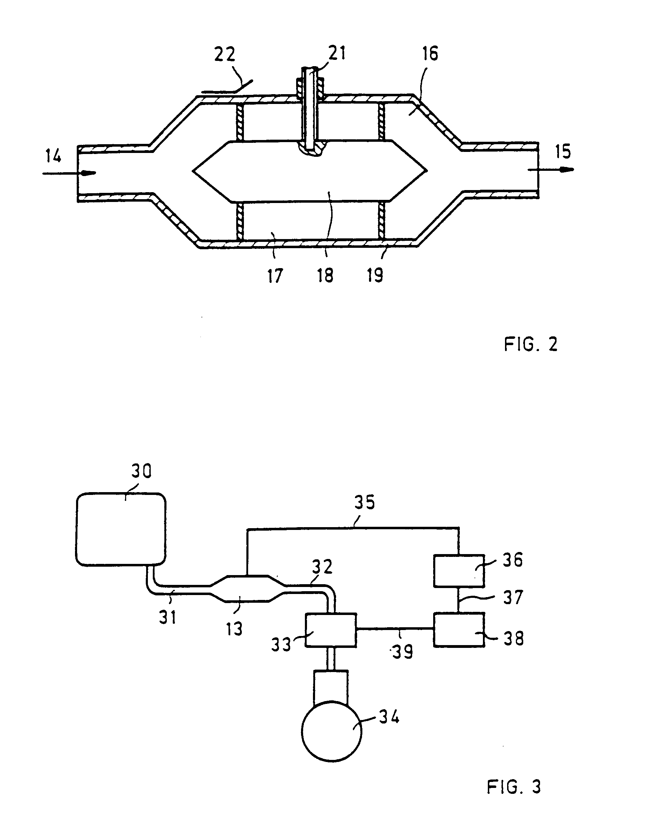

Fig. 2 zeigt eine bevorzugte Ausführungsform einer Meßzelle zur Ausführung des Verfahrens gemäß der Erfindung. In die Meßzelle gelangt der Kraftstoff über Zufluß 14, und er verläßt die Meßzelle über Abfluß 15. Der Kraftstoffstrom teilt sich in der flächenhaften Darstellung der Fig. 2 auf in Strömungswege 16 und 17, die durch einen Mittelzylinder 18 gebildet werden. Wenn der Mittelzylinder 18 und Außenmantel 19 ganz oder teilweise elektrisch leitend sind, können diese Wandungen bzw. Wandungsteile der Meßzelle die Elektroden des Kondensators einer Meß- bzw. Auswerteschaltung darstellen. Innerhalb der wenigstens teilweise leitfähigen Wandung der Meßzelle, die die erste Elektrode darstellt, ist dann der wenigstens teilweise leitfähige Mittelzylinder 18 als Strömungskörper die zweite Elektrode.

Der Mittelzylinder 18 bildet zusammen mit dem Außenmantel 19 den eigentlichen Meßkondensator, der das Meßvolumen umschließt. An Anschlüssen 21 und 22 können die entsprechenden Werte abgegriffen werden.

Wie aus der schematischen Darstellung der Fig. 3 hervorgeht, gelangt mit Alkoholanteilen gemischter Kraftstoff aus Kraftstofftank 30 über eine Leitung 31 zur Meßzelle 13 und von dort über eine Leitung 32 zu einer Zumeßeinrichtung 33, die im Regelfall eine Einspritzpumpe mit entsprechenden Einspritzdüsen ist. Der Kraftstoff wird in direkter oder indirekter Einspritzung in Motor 34 zugeführt.

Die in der Meßzelle 13 abgegriffenen Werte der Kapazität und des Leitwertes werden über Meßleitung 35 einer gemeinsamen Auswerteschaltung oder Auswerteeinheit 36 zugeführt. Insbesondere kann es dabei vorteilhaft sein, daß eine Wechselspannung oder eine von Wechselspannung überlagerte Gleichspannung an zwei Kondensatorelektroden in der Meßzelle angelegt und die Dielektrizitätszahl in Abhängigkeit von dem Real- und/oder Imaginärteil von Kenngrössen des fließenden Stromes bestimmt wird.

Die von der Auswerteschaltung 36 abgegebenen Signale gelangen über eine Leitung 37 zu einem Einspritzrechner 38, und dieser steuert über eine Leitung 39 die Zumeßeinrichtung 33.

Der jeweils in der Meßzelle gemessene und in der Auswerteeinheit 36 ausgewertete Mischungsanteil wird also betrieblich nicht verändert, sondern es wird vielmehr die Art der Einspritzung je nach den gemessenen und ausgewerteten Werten geändert. Gemäß einer bevorzugten Ausführungsform kann bei der Steuerung bzw. Regelung von Einspritzbrennkraftmaschinen die Messung des Alkoholanteils des zugeführten Kraftstoffs zur Vorsteuerung der Einspritzmenge dienen, während die Feinregelung des Luftverhältnisses über eine Lambdaregelung bekannter Art erfolgt. Insbesondere bei Kraftfahrzeugmotoren kann es dabei zweckmäßig sein, daß bei der Anwendung des Verfahrens gemäß der Erfindung auf Einspritzbrennkraftmaschinen die gemeinsame Schaltung als Auswerteeinheit in das Einspritzsystem schaltungs- und programmtechnisch integriert ist. Weitere Vorteile können verfahrenstechnisch dadurch erreicht werden, daß zusätzlich die Temperatur des in der Meßzelle 13 befindlichen Kraftstoffs festgestellt und in die Auswerteeinheit zur Kompensation des Temperatureinflusses eingegeben wird.

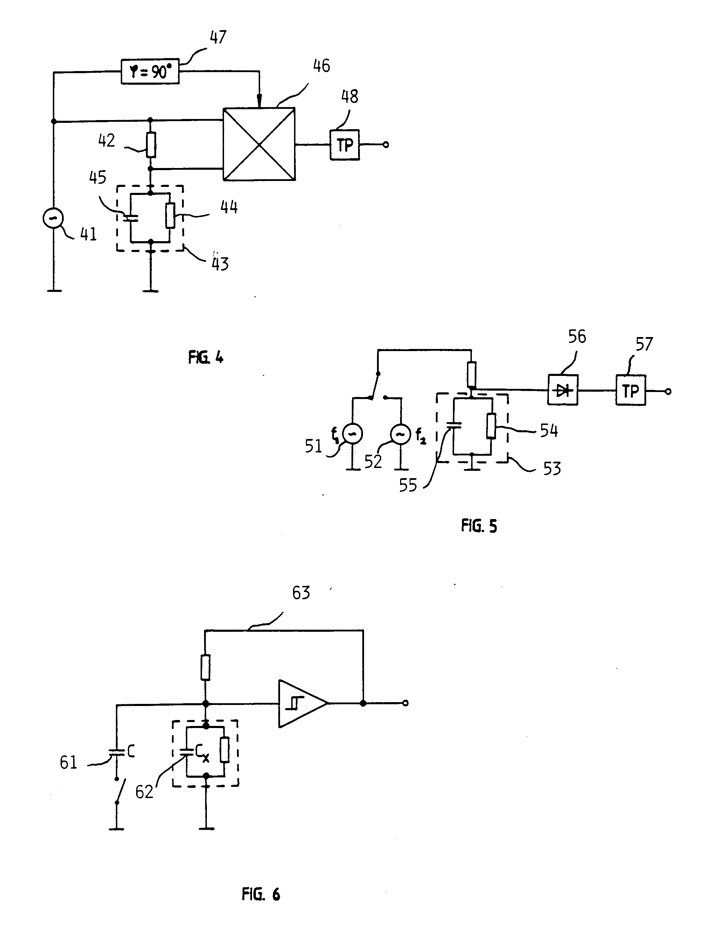

Fig. 4 zeigt eine bevorzugte Ausführungsform einer Schaltung zur Kapazitätsbestimmung. Eine Wechselspannungsquelle 41 beaufschlagt über einen niederohmigen Widerstand 42, der als Stromsensor arbeitet, eine Meßzelle 43 mit einer sinusförmigen Spannung.

Der sich ergebende Stromfluß, der am Widerstand 42 als Spannungsabfall abgegriffen wird, enthält einen Realteil, der nur vom Leitwert 44 der Meßzelle abhängt und einen Imaginärteil, der nur von der Kapazität 45 der Meßzelle abhängt. Zur ausschließlichen Auswertung des Imaginärteils wird der abgegriffene Strom mit einer cosinusförmigen Spannung in einem Multiplizierer 46 multipliziert, die z.B. aus der sinusförmigen Spannung über einen Phasenschieber 47 gewonnen wird. Durch einen Tiefpaßfilter 48 wird der Gleichspannungsanteil der Multiplikatorausgangsspannung extrahiert, der ein Maß für die Kapazität darstellt.

Anstelle einer Multiplikation kann auch eine einfache Synchrongleichrichtung erfolgen.

Eine weitere besonders vorteilhafte Ausführungsform gemäß Fig. 5 ergibt sich, wenn zwei unterschiedliche Frequenzen zweier Spannungserzeuger 51 und 52 zur Messung des Spannungsgabfalls an Meßzelle 53 mit Leitwert 54 und Kapazität 55 verwendet werden. Es wird dann nur der Betrag der über einen Gleichrichter 56 und einen Tiefpaß 57 gebildeten Ausgangsspannung ausgewertet.

Bei einer weiteren Gruppe von bevorzugten Ausführungsformen kann die Kapazität der Meßzelle als das frequenzbestimmende Glied einer Schwingschaltung verwendet werden, wobei der Leitwert einen Quereinfluß bewirkt.

Gemäß Fig. 6 wird in einer bevorzugten Ausführungsform die Messung in zwei Phasen durchgeführt, wobei bei der zweiten Messung eine definierte Kapazität C eines Kondensators 61 zur Meßzellenkapazität Cx der Meßzelle 62 parallel geschaltet wird. Man erhält so zwei unterschiedliche Frequenzen, deren Auswertung eine Aussage über die Kapazität liefert. Im einfachsten Fall ergibt sich die Formel:

$$\frac{f2}{f1} = \frac{Cx}{Cx + C}$$

Besondere Vorteile hinsichtlich der Serienfertigung ergeben sich, wenn anstelle der geschalteten Kapazität eine schaltbare Verzögerung beispielsweise in einen Rückkopplungszweig 63 eingefügt wird. Diese wirkt wie eine Parallelschaltung der Kapazität frequenzerniedrigend, so daß eine gleichartige Auswertung der Frequenzen erfolgen kann.

Eine weitere vorteilhafte Ausführungsform besteht darin, die Messung mit Hilfe einer Meßbrücke durchzuführen. Dabei muß der Brückenabgleich automatisch erfolgen, wobei der für die Kapazität eingestellte Wert als Maß für die Kapazität der Meßzelle ausgewertet wird.

Da der Zusammenhang zwischen Dielektrizitätszahl und Alkoholgehalt stark von der Temperatur abhängt, ist eine Verknüpfung des Kapazitätsmeßwertes mit dem Wert der Kraftstofftemperatur vorteilhaft, um eine von der Temperatur unabhängige Ausgangsgröße als Maß für den Alkoholgehalt zu erzielen. Diese Verknüpfung kann zusammen mit einer Linearisierung der Ausgangsspannung mit Hilfe eines Prozessors erfolgen. Besonders vorteilhaft ist dabei die Verwendung des normalerweise für die Einspritzung oder Zündung verwendeten Prozessors.

Für eine weite Anwendung auf dem Gebiet der Verbrennungskraftmaschinen ist es auch vorteilhaft, wenn die Verknüpfung der gemessenen Größen und die Linearisierung schon im Sensor erfolgt, und zwar ohne Prozessor durch eine möglichst einfache und kostengünstige Schaltung. Hierzu werden die Werte für die Temperatur und die Kapazität als Digitalgrößen auf die Adressleitungen eines programmierbaren Bausteines (z.B. eines ROM, EPROM, PAL) geführt. An den Datenleitungen des entsprechend programmierten EPROMS o.ä. kann dann die Information über den Alkoholgehalt in digitaler Form abgenommen werden und ggf. noch einem D/A-Wandler zur Umsetzung in einen Analogwert zugeführt werden.

Eine der Ausgangsleitungen kann auch zur Ausgabe eines Fehlersignals verwendet werden, das Bereichsüberschreitungen in den Eingangsdaten und somit Fehlfunktionen der Sensoren feststellen kann.

Eine weitere bevorzugte Ausführungsform wird durch Verwendung einer Frequenz oder eines Frequenzbereiches erzielt, in der bzw. dem durch den Ausfall bestimmter Polarisationsmechanismen Überhöhungen der Dielektrizitätszahl kurz vor der Ausfallfrequenz meßtechnisch ausgenutzt werden.

**Patentansprüche**

1. Verfahren zur Feststellung des Alkoholgehaltes und/oder des Heizwertes von Kraftstoffen durch Messung elektrisch meßbarer Größen in einer den Kraftstoff enthaltenden Meßzelle zur Bestimmung der Dielektrizitätszahl des in der Zelle enthaltenen Gemisches als Kenngröße des Alkoholgehaltes bzw. des Heizwertes, durch Messung der Kapazität eines in der Meßzelle gebildeten Kondensators, dessen Dielektrikum durch in der Meßzelle enthaltenen Kraftstoff gebildet wird, und die Feststellung der Kapazität schaltungstechnisch und/oder rechnerisch unabhängig von dem Einfluß der Leitfähigkeit des Kraftstoffs durch Anlegen einer Wechselspannung oder einer von Wechselspannung überlagerter Gleichspannung an zwei Kondensatorelektroden in der Meßzelle, womit die Dielektrizitätszahl in Abhängigkeit von dem Real- und/oder Imaginärteil von Kenngrößen des fließenden Stromes bestimmt wird, und wobei die Temperatur des Kraftstoffes als Korrekturgröße ausgewertet wird.

2. Verfahren nach Anspruch 1, dadurch gekennzeichnet, daß die Messung der Kapazität durch Beaufschlagen der Kondensatorelektroden in der den Kraftstoff enthaltenden Meßzelle mit einer sinusförmigen Spannung und Auswertung des Imaginärteils des Stromes erfolgt.

3. Verfahren nach Anspruch 1 oder 2, dadurch gekennzeichnet, daß die Auswertung des Imaginärteils des Stromes durch Synchrongleichrichtung erfolgt,

4. Verfahren nach Anspruch 1 oder 2, dadurch gekennzeichnet, daß die Auswertung des Imaginärteils des Stromes durch Multiplikation mit einem cosinus-förmigen Signal erfolgt.

5. Verfahren nach Anspruch 1, dadurch gekennzeichnet, daß eine Auswertung des Spannungsabfalls des Stromes durch die Meßzelle nur hinsichtlich des Betrages bei zwei unterschiedlichen Frequenzen erfolgt.

6. Verfahren nach Anspruch 1, dadurch gekennzeichnet, daß die Kapazität in einer schwingfähigen Schaltung gemessen wird, in der die Kraftstoffmenge in der Meßzelle Teil eines Kondensators ist,

7. Verfahren nach Anspruch 1, gekennzeichnet durch ein Meßverfahren, bei dem die Meßzelle als frequenzbestimmendes Glied in einem Generator verwendet wird, dessen Frequenz durch Parallelschaltung eines bekannten Kondensators geändert wird, wobei die beiden unterschiedlichen Frequenzen zur Bestimmung der Kapazität ausgewertet werden.

8. Verfahren nach Anspruch 7, dadurch gekennzeichnet, daß die Frequenzänderung durch ein schaltbares Verzögerungsglied erreicht wird.

9. Verfahren nach Anspruch 1, dadurch gekennzeichnet, daß die Messung der Kapazität über eine Meßbrücke erfolgt, die automatisch abgeglichen wird.

10. Verfahren nach einem der Ansprüche 1 bis 9, dadurch gekennzeichnet, daß eine kennfeldmäßige Auswertung ohne Anwendung eines Prozessors unmittelbar in einem ROM erfolgt, indem die digitalisierten Werte als Adresse benutzt werden und der Zustand der Datenleitungen für die Bestimmung der Dielektrizitätszahl ausgewertet wird.

11. Verfahren nach einem der Ansprüche 1 bis 10, dadurch gekennzeichnet, daß die Messung der elektrisch meßbaren Größen in einer Schaltung erfolgt, die in unmittelbarer Nähe der Meßzelle angebracht ist, während die Weiterverarbeitung der Signale in einer weiter entfernten Schaltung erfolgt.

12. Verfahren nach einem der Ansprüche 1 bis 11, dadurch gekennzeichnet, daß alle oder ein Teil der gemessenen Größen in einem Signal zusammengefaßt werden, das über eine Signalleitung der Weiterverarbeitungsschaltung zugeführt wird.

13. Verfahren nach einem der Ansprüche 1 bis 12, dadurch gekennzeichnet, daß die Meßfrequenz auch bei sich ändernder Kapazität konstant gehalten wird.

14. Verfahren nach Anspruch 13, dadurch gekennzeichnet, daß die Regelung der Frequenz durch eine "Switched-Capacitor"-Schaltung erfolgt.

15. Verfahren nach einem der Ansprüche 1 bis 14, dadurch gekennzeichnet, daß mindestens eine Frequenz, die zur Messung verwendet wird, im Bereich einer Überhöhung der Dielektrizitätszahl eines der beteiligten Stoffe liegt.

16. Verfahren nach einem der Ansprüche 1 bis 15, dadurch gekennzeichnet, daß bei Anwendung auf die Steuerung bzw. Regelung einer Einspritzbrennkraftmaschine die Messung des Alkoholanteils des zugeführten Kraftstoffs zur Vorsteuerung der Einspritzmenge dient, während die Feinregulierung des Luftverhältnisses über eine Lambdaregelung bekannter Art erfolgt.

17. Verfahren nach einem der Ansprühe 1 bis 16, dadurch gekennzeichnet, daß Wandungen der Meßzelle als Elektroden eines Kondensators der Auswerteschaltung verwendet werden.

**18.** Verfahren nach Anspruch 16 oder 17, dadurch gekennzeichnet, daß bei der Anwendung auf Einspritzbrennkraftmaschinen die gemeinsame Schaltung und/oder die Auswertung der Meßsignale in das Einspritzsystem schaltungs- und programmtechnisch integriert wird.

## Claims

**1.** A method for determining the alcohol content and/or the calorific value of fuels by measuring electrically measurable variables in a measuring cell containing the fuel for determining the dielectric constant of the mixture contained in the cell as a characteristic of the alcohol content or the calorific value by measuring the capacitance of a capacitor formed in the measuring cell, the dielectric of which is formed by fuel contained in the measuring cell, and the determination of the capacitance by means of the circuit design and/or by computation independently of the influence of the conductivity of the fuel by applying an alternating voltage or a direct-current voltage with a superposed alternating voltage to two capacitor electrodes in the measuring cell, by means of which the dielectric constant is determined depending on the real and/or imaginary part of characteristics of the flowing current, and the temperature of the fuel being evaluated as a correcting quantity.

**2.** A method according to Claim 1, characterised in that the measurement of the capacitance takes place by supplying the capacitor electrodes in the measuring cell containing the fuel with a sinusoidal voltage and the evaluation of the imaginary part of the current.

**3.** A method according to Claim 1 or 2, characterised in that the evaluation of the imaginary part of the current takes place by synchronous rectification.

**4.** A method according to Claim 1 or 2, characterised in that the evaluation of the imaginary part of the current takes place by multiplication with a cosinusoidal signal.

**5.** A method according to Claim 1, characterised in that evaluation of the voltage drop of the current through the measuring cell takes place only with respect to the amount for two different frequencies.

**6.** A method according to Claim 1, characterised in that the capacitance is measured in an oscillatory circuit, in which the amount of fuel in the measuring cell forms part of a capacitor.

**7.** A method according to Claim 1, characterised by a measuring method in which the measuring cell is used as the frequency-determining element in a generator, the frequency of which is changed by the parallel connection of a known capacitor, the two different frequencies being evaluated to determine the capacitance.

**8.** A method according to Claim 7, characterised in that the change in frequency is achieved by a switchable delay element.

**9.** A method according to Claim 1, characterised in that the measurement of the capacitance takes place by means of a measuring bridge which is automatically adjusted.

**10.** A method according to one of Claims 1 to 9, characterised in that an evaluation of performance characteristics without the use of a processor takes place directly in a ROM, in that the digitised values are used as an address and the state of the data lines is evaluated for determining the dielectric constant.

**11.** A method according to one of Claims 1 to 10, characterised in that the measurement of the electrically measurable values takes place in a circuit which is applied in the immediate vicinity of the measuring cell, whereas the further processing of the signals takes place in a circuit further removed.

**12.** A method according to one of Claims 1 to 11, characterised in that all or a part of the measured variables are combined in a signal which is fed to the further-processing circuit via a signal line.

**13.** A method according to one of Claims 1 to 12, characterised in that the measuring frequency is kept constant even if the capacitance changes.

**14.** A method according to Claim 13, characterised in that the regulation of the frequency takes place by a "switched-capacitor" circuit.

**15.** A method according to one of Claims 1 to 14, characterised in that at least one frequency which is used for measurement lies in the region of an increase of the dielectric constant of one of the participating substances.

**16.** A method according to one of Claims 1 to 15, characterised in that when applied to the control or regulation of a fuel-injection internal combustion engine, the measurement of the alcohol content of the fuel supplied serves for preliminary control of the injection quantity, whereas the fine adjustment of the air ratio takes place by means of a lambda control of known type.

17. A method according to one of Claims 1 to 16, characterised in that walls of the measuring cell are used as electrodes of a capacitor of the evaluation circuit.

18. A method according to Claim 16 or 17, characterised in that when applied to fuel-injection internal combustion engines the common circuit and/or the evaluation of the measuring signals is integrated by means of the circuit and program design into the fuel-injection system.

**Revendications**

1. Procédé de détermination de la teneur en alcool et/ou du pouvoir calorifique de carburants par mesure de grandeurs électriquement mesurables dans une cellule de mesure contenant le carburant pour déterminer la constante diélectrique du mélange contenu dans la cellule en tant que grandeur caractéristique de la teneur en alcool ou du pouvoir calorifique, par mesure de la capacité d'un condensateur qui est formé dans la cellule de mesure et dont le diélectrique est formé par le carburant contenu dans la cellule de mesure, et par détermination de la capacité par technique de circuit et/ou par le calcul, indépendamment de l'influence de la conductibilité du carburant, par application d'une tension alternative, ou d'une tension continue à laquelle se superpose une tension alternative, à deux électrodes de condensateur de la cellule de mesure, ce par quoi on détermine la constante diélectrique en fonction de la partie réelle et/ou de la partie imaginaire des grandeurs caractéristiques du courant qui passe, la température du carburant étant traitée en tant que grandeur correctrice.

2. Procédé selon la revendication 1, caractérisé par le fait que la mesure de la capacité se fait en soumettant les électrodes du condensateur qui sont dans la cellule de mesure contenant le carburant à une tension sinusoïdale et en traitant la partie imaginaire du courant.

3. Procédé selon la revendication 1 ou 2, caractérisé par le fait que le traitement de la partie imaginaire du courant se fait par redressement synchrone.

4. Procédé selon la revendication 1 ou 2, caractérisé par le fait que le traitement de la partie imaginaire du courant se fait par multiplication par un signal cosinusoïdal.

5. Procédé selon la revendication 1, caractérisé par le fait qu'un traitement de la chute de tension du courant à travers la cellule de mesure ne se fait qu'en ce qui concerne sa valeur dans le cas de deux fréquences différentes.

6. Procédé selon la revendication 1, caractérisé par le fait que l'on mesure la capacité dans un circuit oscillant dans lequel le débit de carburant dans la cellule de mesure fait partie d'un condensateur.

7. Procédé selon la revendication 1, caractérisé par un procédé de mesure dans le cas duquel on emploie la cellule de mesure en tant qu'organe qui détermine la fréquence dans un générateur et dont on modifie la fréquence par mise en circuit en parallèle d'un condensateur connu, les deux fréquences différentes étant traitées pour déterminer la capacité.

8. Procédé selon la revendication 7, caractérisé par le fait que la modification de fréquence s'obtient au moyen d'un élément temporisateur commutable.

9. Procédé selon la revendication 1, caractérisé par le fait que la mesure de la capacité se fait au moyen d'un pont de mesure qui se compense automatiquement.

10. Procédé selon l'une des revendications 1 à 9, caractérisé par le fait qu'un traitement à base d'un réseau de caractéristiques se fait immédiatement dans une mémoire ROM sans utilisation d'un processeur, en ce sens que l'on utilise les valeurs numérisées en tant qu'adresses et que l'on exploite l'état des lignes de données pour déterminer la constante diélectrique.

11. Procédé selon l'une des revendications 1 à 10, caractérisé par le fait que la mesure des grandeurs mesurables électriquement se fait dans un circuit qui est appliqué au voisinage immédiat de la cellule de mesure tandis que la poursuite du traitement des signaux se fait dans un circuit plus éloigné.

12. Procédé selon l'une des revendications 1 à 11, caractérisé par le fait que l'on regroupe la totalité ou une partie des grandeurs mesurées dans un signal que l'on amène, par une ligne de signaux, au circuit de poursuite du traitement.

**13.** Procédé selon l'une des revendications 1 à 12, caractérisé par le fait que l'on maintient constante la fréquence de mesure même si la capacité se modifie.

**14.** Procédé selon la revendication 13, caractérisé par le fait que la régulation de la fréquence se fait au moyen d'un circuit à "capacités commutées".

**15.** Procédé selon l'une des revendications 1 à 14, caractérisé par le fait qu'au moins une fréquence que l'on emploie pour la mesure se situe dans le domaine d'une forte augmentation de la constante diélectrique de l'un des produits concernés.

**16.** Procédé selon l'une des revendications 1 à 15, caractérisé par le fait que lorsqu'on l'applique à la commande ou à la régulation d'un moteur à combustion interne à injection, la mesure de la teneur en alcool du carburant amené sert à une précommande du débit d'injection tandis que la régulation fine de la proportion d'air se fait au moyen d'une régulation lambda, de type connu.

**17.** Procédé selon l'une des revendications 1 à 16, caractérisé par le fait que l'on emploie des parois de la cellule de mesure comme électrodes d'un condensateur du circuit de traitement.

**18.** Procédé selon la revendication 16 ou 17, caractérisé par le fait que lors de l'application sur des moteurs à combustion interne à injection, on intègre dans le système d'injection, au point de vue technique de circuit et de programmation, le circuit commun et/ou le traitement commun des signaux de mesure.

FIG. 1

FIG. 2

FIG. 3

FIG. 4

FIG. 5

FIG. 6